# EUROPEAN PATENT APPLICATION

(11) **EP 1 669 066 A1**
(43) Date of publication of application: **14.06.2006**
(21) Application number: 04792178.8
(22) Date of filing: 01.10.2004
(51) Int. Cl.: A61K 31/20, A61K 9/08, A61K 47/02, A61P 25/28

(54) **INFUSION PREPARATION CONTAINING (2R)-2-PROPYLOCTANOIC ACID AS THE ACTIVE INGREDIENT**

(30) Priority: 03.10.2003 JP 2003345125
(71) Applicant: ONO PHARMACEUTICAL CO., LTD., Osaka-shi, Osaka 541-8526 (JP)
(72) Inventor: SUDOH, Masao, Ono Pharmaceutical Co., Ltd., Mishima-gun, Osaka 618-8585 (JP); TANIKAWA, Seiichi, Ono Pharmaceutical Co., Ltd., Mishima-gun, Osaka 618-8585 (JP)
(74) Representative: Henkel, Feiler & Hänzel
(86) International application number: PCT/JP2004/014896
(87) International publication number: WO 2005/032538

(57) **Abstract**

The present invention relates to an infusion preparation which is useful for neurodegenerative diseases, which comprises (2R)-2-propyloctanoic acid or a salt thereof and a basic metal ion which is supplied from a metal salt or a metal hydroxide of weak acid, preferably about 1 to about 5 equivalents of a basic metal ion based on 1 equivalent of (2R)-2-propyloctanoic acid or a salt thereof, and optionally further comprises other infusion components. The infusion preparation of the present invention is has a pH appropriate for intravenous administration, and is useful for intravenous administration since it does not require any preparative operation such as dissolution, dilution or the like at the time of use.

## Description

### Technical Field

The present invention relates to an infusion preparation for continuous intravenous administration containing (2R)-2-propyloctanoic acid or a salt thereof, which is useful as an agent for treating and/or preventing neurodegenerative diseases including cerebral infarction, and a process for producing the same.

### Background Art

Patients with cerebral stroke in general are often taken to hospitals only after they fall unconscious due to cerebral infarction or cerebral hemorrhage, and such a patient with cerebral stroke receives treatment unavoidably while remaining unconscious. Therefore, a medicament administered for the treatment of such a disease is preferably a preparation suitable for parenteral administration such as an injection. Particularly, in an expectation of rapid expression of the effect, it is preferably an injection for intravenous administration. At present, Radicut (edaravone) and t-PA, which are used as agents for treating cerebral infarction, are all injections for intravenous administration.

However, upon use of an injection which is provided in the form of vials, ampoules, prefilled syringes or the like, apart from the case where an injectable solution having a high concentration is directly injected to a patient intravenously, when the injection is to be once diluted in a medium such as an infusion solution, and then continuously injected by means of an intravenous drip, manipulation such as injection thereof into the infusion solution by dissolving a plurality of vials in the medium in accordance with dosage or the like is necessary, and thus such action is quite troublesome to a medical worker. Furthermore, these actions may also result in medical malpractice such as making a mistake in preparation of the dosage and subsequently worsening the condition of the patient, or the like. Therefore, a pharmaceutical preparation injected by means of the intravenous drip is preferably provided in the state of being preliminarily dissolved in the infusion solution, that is, as an infusion preparation.

Meanwhile, as examples of an injection comprising (2R)-2-propyloctanoic acid or a salt thereof, which is useful as an agent for treating or preventing various cerebral nerve diseases including cerebral stroke, the injections described in the following have been reported.

A pentanoic acid derivative which is useful as a medicament for cerebral function improvement is known to be used as an injection, upon mixing with at least one inert aqueous diluting agent (distilled water for injection, saline, *etc.)* or at least one inert non-aqueous diluting agent (propylene glycol, polyethylene glycol, *etc*.) (see, for example, the specifications of EP 0632008 and EP 1174131). These publications describe that an injection can further comprise auxiliaries such as a preservative, a wetting agent, an emulsifier, a dispersing agent, a stabilizing agent and a solubilizing aid.

However, there has been no specific report hitherto on an infusion preparation for continuous intravenous administration, comprising (2R)-2-propyloctanoic acid or a salt thereof as preliminarily dissolved in the infusion solution.

### Disclosure of the Invention

The present inventors have conducted an investigation in various ways using (2R)-2-propyloctanoic acid, and as a result, they found that it is preferable to administer the compound, not by bolus administration, but in the form of a dilution, that is, via continuous intravenous administration by means of an intravenous drip.

Thus, the purpose of the present invention is to provide an infusion preparation for continuous intravenous administration comprising (2R)-2-propyloctanoic acid or a salt thereof, which has a pH appropriate for intravenous administration, and which does not require any preparative operation such as dissolution or dilution at the time of use, and a process for producing the same.

The inventors have conducted an extensive investigation, and as a result, they have found that (2R)-2-propyloctanoic acid or a salt thereof, which is useful as a therapeutic agent or a prophylactic agent for various cerebral nerve diseases including cerebral stroke, can be prepared alone or dissolved in an aqueous solvent according to the respective corresponding solubility and prepared as an infusion preparation for continuous intravenous administration therefrom. However, the infusion preparation comprising (2R)-2-propyloctanoic acid or a salt thereof prepared in this way was found to undergo clouding with slight fluctuations of pH, for example, a shift to the acidic side. As such, there are problems in actually applying to a patient an infusion preparation which is sensitive to the fluctuations of the solution pH, leading to a change like clouding. That is, there is in fact the risk of clouding of the preparation, upon preparing an injection which can be administered to a patient, by direct manipulation of a medical worker, for combined use with other agents, or during the action of intravascular administration to a patient. The inventors have conducted a further investigation, and as a result, they have found that it is possible to provide an infusion preparation for continuous intravenous administration which has a pH appropriate for intravenous administration and is resistant to fluctuations of pH, by allowing the coexistence of basic metal ions by addition of a certain amount of a metal salt of weak acid such as trisodium phosphate or sodium carbonate. The inventors have conducted a more specific research based on this founding to accomplish the present invention.

That is, the present invention relates to the followings and the like:
(1) An infusion preparation comprising (2R)-2-propyloctanoic acid or a salt thereof and a basic metal ion.
(2) The infusion preparation according to the above (1), which comprises at least one selected from a metal salt of phosphoric acid, a metal salt of carbonic acid, a metal salt of sulfurous acid, a metal salt of organic sulfonic acid and a metal salt of organic C2-6 carboxylic acid as a source of the basic metal ion, and optionally further comprises a metal hydroxide.
(3) The infusion preparation according to the above (1), which further comprises one or at least two selected from (i) electrolytes, (ii) saccharides, (iii) vitamins and (iv) protein amino acids.
(4) The infusion preparation according to the above (1), which comprises about 1 to about 5 equivalents of the basic metal ion based on 1 equivalent of (2R)-2-propyloctanoic acid or a salt thereof
(5) The infusion preparation according to the above (2), which comprises at least one selected from trisodium phosphate, disodium hydrogen phosphate, sodium dihydrogen phosphate, sodium carbonate, sodium hydrogen carbonate, sodium sulfite, sodium hydrogen sulfite, tripotassium phosphate, dipotassium hydrogen phosphate, potassium dihydrogen phosphate, potassium carbonate, potassium hydrogen carbonate, potassium sulfite and potassium hydrogen sulfite, and optionally further comprises sodium hydroxide and/or potassium hydroxide, as a source(s) of the basic metal ion.
(6) The infusion preparation according to the above (2), which comprises sodium hydroxide and/or potassium hydroxide, and further comprises at least one selected from disodium hydrogen phosphate, sodium dihydrogen phosphate, sodium hydrogen carbonate, sodium hydrogen sulfite, dipotassium hydrogen phosphate, potassium dihydrogen phosphate, potassium hydrogen carbonate and potassium hydrogen sulfite, as sources of the basic metal ion.
(7) The infusion preparation according to the above (1), which has a pH of about 5.0 to about 9.0.
(8) The infusion preparation according to the above (1), which comprises about 0.1 to about 20 mg of (2R)-2-propyloctanoic acid or a salt thereof per mL.
(9) A container for infusion which is filled with the infusion preparation depicted in the above (8) at about 50 mL, about 100 mL, about 200 mL, about 250 mL, about 500 mL or about 1,000 mL per one unit.
(10) The infusion preparation according to the above (1), which comprises about 1 to about 5 equivalents of the basic sodium ion based on 1 equivalent of (2R)-2-propyloctanoic acid or a salt thereof; comprises at least one selected from a sodium salt of phosphoric acid and a sodium salt of carbonic acid as a source of the basic sodium ion, and optionally further comprises sodium hydroxide, as a source(s) of the basic sodium ion; and has a pH of about 5.0 to about 9.0.
(11) The infusion preparation according to the above (10), which further comprises 0.9% (w/v) sodium chloride.
(12) The infusion preparation according to the above (1), wherein the salt of (2R)-2-propyloctanoic acid is a sodium salt or a basic natural amino acid salt.
(13) The infusion preparation according to the above (1), which is an agent for preventing and/or treating neurodegenerative diseases, nerve disorders or diseases in need of nerve regeneration.
(14) A process for producing an infusion preparation comprising (2R)-2-propyloctanoic acid or a salt thereof and a basic metal ion, which comprises dissolving (2R)-2-propyloctanoic acid or a salt thereof, one or at least two selected from a metal salt of phosphoric acid, a metal salt of carbonic acid, a metal salt of sulfurous acid, a metal salt of organic sulfonic acid and a metal salt of C2-6 organic acid, and optionally metal hydroxide in an aqueous medium to thereby prepare a solution comprising about 2.5 to about 100 mg/mL of(2R)-2-propyloctanoic acid or a salt thereof and having a pH of about 8.4 to about 9.0; diluting the prepared solution with one or at least two selected from (i) electrolytes, (ii) saccharides, (iii) vitamins and (iv) protein amino acids to thereby adjust the concentration of (2R)-2-propyloctanoic acid or a salt thereof in the solution to about 0.1 to about 20 mg/mL; and filling a container for infusion with the diluted solution.
(15) A method for preventing and/or treating neurodegenerative diseases, nerve disorders or diseases in need of nerve regeneration, which comprises administering an effective amount of the infusion preparation according to the above (1) to a mammal.
(16) Use of the infusion preparation according to the above (1) for the manufacture of an agent for preventing and/or treating neurodegenerative diseases, nerve disorders or diseases in need of nerve regeneration.

(2R)-2-Propyloctanoic acid used in the present invention is a compound represented by formula (I): 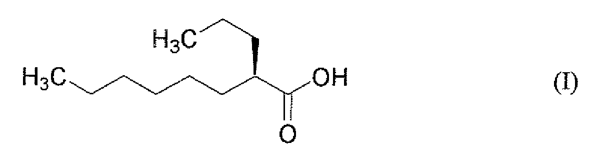 wherein 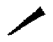 represents β-configuration.

The salt of (2R)-2-propyloctanoic acid is preferably a pharmaceutically acceptable salt. The pharmaceutically acceptable salt is preferably a non-toxic water-soluble salt. Examples of suitable salt of (2R)-2-propyloctanoic acid, for example, include salts with inorganic bases, salts with organic bases, salts with basic natural amino acids and the like. The salt with an inorganic base is preferably, for example, an alkali metal salt (for example, a sodium salt, a potassium salt, a lithium salt, *etc*.), an ammonium salt (for example, a tetramethylammonium salt, a tetrabutylammonium salt, *etc*.), or the like. The salt with an organic base is preferably, for example, a salt with alkylamine (for example, methylamine, dimethylamine, trimethylamine, triethylamine, etc.), heterocyclic amine (for example, pyridine, picoline, piperidine, *etc*.), alkanolamine (for example, ethanolamine, diethanolamine, triethanolamine, *etc*.), dicyclohexylamine, N,N¹-dibenzylethylenediamine, cyclopentylamine, benzylamine, phenethylamine, tris(hydroxyrnethyl)methylamine, N-methyl-D-glucamine or the like. The salt with a basic natural amino acid is not particularly limited, so long as it is a salt with a basic amino acid which is naturally present and can be purified, and it is preferably, for example, a salt with arginine, lysine, ornithine, histidine or the like. Among these, more preferred are, for example, an alkali metal salt or a basic natural amino acid salt, and particularly preferred is a sodium salt.

In the present invention, (2R)-2-propyloctanoic acid or a salt thereof is not limited to those which are substantially pure single substances, and they may include impurities (for example, byproducts, solvents, starting materials, etc. which originate from the preparation process, or decomposition products, *etc*.) within the scope acceptable in an active ingredient for a medicament. The contents of the impurities acceptable in an active ingredient for a medicament may depend on the impurities contained, but the contents are preferably, for example, about 20 ppm or less for heavy metals, about 1.49% by weight or less for the (S)-isomer which is an optical isomer, about 5,000 ppm or less in total for residual solvents such as 2-propanol or heptane, and about 0.2% by weight or less for water, respectively.

(2R)-2-Propyloctanoic acid or a salt thereof can be prepared by methods known per se, for example, the methods described in the specification of EP 0632008, the pamphlet of WO 99158513, the pamphlet of WO 00/48982, the specification of JP 3032447 (registration number), the specification of JP 3084345 (registration number) and the like, or by appropriate combinations of such methods.

The infusion preparation of the present invention includes all of liquid preparations for continuous injection into the blood vessel, preferably veins. Such infusion preparation may be, for example, an aqueous infusion preparation in which the medium is substantially water, or a water-in-oil emulsion type infusion preparation such as a fatty fluid, which is used in intravenous alimentation methods such as a hyperalimentation method and a total parenteral nutrition method. Preferred as the infusion preparation of the present invention is an aqueous infusion preparation which can be intravenously administered through peripheral veins.

The infusion preparation of the present invention comprises a basic metal ion in addition to (2R)-2-propyloctanoic acid or a salt thereof which is the active ingredient.

In the specification, the term "basic metal ion" means the metal ion supplied by a metal compound in an aqueous solution, and preferably the metal ion supplied by a metal compound which is basic in an aqueous solution. The metal compound which serves as the source of the basic metal ion is not particularly limited, and includes, for example, a metal salt of weak acid, metal hydroxide and the like. The weak acid which constitutes a metal salt of weak acid may be organic or inorganic acid, or even polyvalent acid. The weak acid includes, for example, phosphoric acid, carbonic acid, boric acid, sulfurous acid, organic sulfonic acid, C2-6 organic carboxylic acids (for example, mono- to trivalent C2-6 organic carboxylic acids, i.e., C2-6 aliphatic monocarboxylic acids, dicarboxylic acids or tricarboxylic acids, *etc*.), and other organic acids.

The metal salt of weak acid of the present invention includes, for example, the salts formed by those weak acids with monovalent alkali metals (for example, sodium, potassium, lithium, rubidium, cesium, francium, *etc*.). As the monovalent alkali metal, for example, sodium, potassium, lithium and the like are preferred, with sodium and potassium being more preferred. Particularly preferred is sodium. The metal salt of weak acid according to the present invention includes, for example, metal salts of phosphoric acid: preferably disodium hydrogen phosphate, sodium dihydrogen phosphate, trisodium phosphate, dipotassium hydrogen phosphate, potassium dihydrogen phosphate, tripotassium phosphate and the like; metal salts of carbonic acid: preferably sodium carbonate, sodium hydrogen carbonate, potassium carbonate, potassium hydrogen carbonate and the like; metal salts of boric acid: preferably sodium borate, potassium borate and the like; metal salts of sulfurous acid: preferably sodium sulfite, sodium hydrogen sulfite, potassium sulfite, potassium hydrogen sulfite and the like; metal salts of organic sulfonic acid: preferably sodium camphorsulfonate, sodium methanesulfonate, sodium ethanesulfonate, sodium trifluoromethanesulfonate, sodium toluenesulfonate, sodium naphthalenesulfonate, sodium 2-hydroxyethanesulfonate, sodium aminoethanesulfonate, potassium camphorsulfonate, potassium methanesulfonate, potassium ethanesulfonate, potassium trifluoromethanesulfonate, potassium toluenesulfonate, potassium naphthalenesulfonate, potassium 2-hydroxyethanesulfonate, potassium aminoethanesulfonate and the like; and metal salts of C2-6 organic carboxylic acids: preferably sodium acetate, sodium propionate, sodium valerate, sodium oxalate, sodium ascorbate, sodium lactate, sodium succinate, sodium citrate, disodium citrate, sodium malate, sodium tartrate, sodium maleate, sodium fumarate, sodium aminoacetate, potassium acetate, potassium propionate, potassium valerate, potassium oxalate, potassium ascorbate, potassium lactate, potassium succinate, potassium citrate, disodium citrate, potassium malate, potassium tartrate, potassium maleate, potassium fumarate, potassium aminoacetate and the like. In addition to these, use can be also made of, for example, sodium aspartate, sodium glutamate, sodium acetyltryptophan, sodium caprylate, sodium gluconate, sodium salitylate, sodium diethylenetriamine pentaacetate, sodium thioglycolate, potassium thiocyanate, sodium thiosulfate, sodium deoxycholate, potassium pyrosulfite, sodium pyrosulfite, sodium methanesulfobenzoate, sodium benzoate, sodium pyrrolinate, potassium aspartate, potassium glutamate, potassium acetyltryptophan, potassium caprylate, potassium gluconate, potassium salitylate, potassium diethylenetriamine pentaacetate, potassium thioglycolate, sodium thiocyanate, potassium thiosulfate, potassium deoxycholate, potassium methanesulfobenzoate, potassium benzoate, potassium pyrrolinate and the like.

The metal salt of weak acid according to the present invention preferably includes, for example, a metal salt of phosphoric acid, a metal salt of carbonic acid, a metal salt of sulfurous acid, a metal salt of organic sulfonic acid, a metal salt of C2-6 organic carboxylic acid or the like, and more preferably for example, metal salts of phosphoric acid, carbonic acid, sulfurous acid or the like. In particular, the metal salts of phosphoric acid (for example, trisodium phosphate, disodium hydrogen phosphate, sodium dihydrogen phosphate, tripotassium phosphate, dipotassium hydrogen phosphate, potassium dihydrogen phosphate, *etc*.) are preferred, with trisodium phosphate or disodium hydrogen phosphate being particularly preferred.

Such a metal salt of weak acid can be incorporated into the infusion preparation of the present invention, not only in their anhydrous form but also in the form of solvate such as hydrate (for example, in the case of metal phosphate, in particular sodium phosphate, trisodium phosphate·dodecahydrate, sodium dihydrogen phosphate dihydrate, disodium hydrogen phosphate dodecahydrate, *etc*.). The metal salt of weak acid is mixed in the solution state or in the solid sate. Furthermore, the metal salts of weak acids may be used in combination of two or more components, if necessary. Among the metal salts of weak acids, preferred is a metal salt having its pKa value in the basic pH range. In the case of metal salts of polyvalent weak acids, preferred is a metal salt having at least one of its plural pKa values in the basic pH range.

In the specification of the present invention, the metal hydroxide includes, for example, the hydroxides of the monovalent alkali metals described above. Specific examples include sodium hydroxide, potassium hydroxide, lithium hydroxide, rubidium hydroxide, cesium hydroxide, francium hydroxide and the like, and among these, preferred are, for example, sodium hydroxide, potassium hydroxide, lithium hydroxide and the like, with sodium hydroxide or potassium hydroxide being more preferred. Particularly preferred is sodium hydroxide. Such metal hydroxide is mixed in the solution state or in the solid state. Furthermore, the metal hydroxide may be used in combination of two or more compounds, if necessary.

In the infusion preparation of the present invention, the metal salt of weak acid added as the source of the basic metal ion also functions as a buffering agent. It is possible to prepare the infusion preparation of the present invention which is resistant to change of the pH, by means of the metal salt of weak acid since it is dissociated in the infusion preparation, and interacts with metal hydroxide, or (2R)-2-propyloctanoic acid or a salt thereof. Specifically, for example, in an aqueous infusion preparation comprising (2R)-2-propyloctanoic acid and trisodium phosphate, the (2R)-2-propyloctanoic acid is converted to (2R)-2-propyloctanoic acid·sodium salt by means of sodium ions donated by trisodium phosphate. Furthermore, trisodium phosphate is converted, as it releases sodium ions in the aqueous solution, to disodium hydrogen phosphate and further to sodium dihydrogen phosphate. When such substances co-exist in equilibrium in the aqueous solution, the infusion preparation of the present invention attains the buffering ability. In another embodiment, for example, in an aqueous infusion preparation comprising (2R)-2-propyloctanoic acid and disodium hydrogen phosphate as well as sodium hydroxide, the (2R)-2-propyloctanoic acid is converted to (2R)-2-propyloctanoic acid·sodium salt by means of sodium ions donated by sodium hydroxide and/or disodium hydrogen phosphate. Sodium hydroxide and/or disodium hydrogen phosphate, as they exchange sodium ions in the aqueous solution, are placed in the state where trisodium phosphate is added, that is, the state wherein disodium hydrogen phosphate and sodium dihydrogen phosphate co-exist in the aqueous solution, or in a state approximating the former state, and come to exist in equilibrium, and thus to exhibit the buffering ability. Thus, with regard to the infusion preparation of the present invention, the metal salt of weak acid interacts with metal hydroxide, or (2R)-2-propyloctanoic acid or a salt thereof and exhibits the same effect as in the case of adding a monobasic phosphate-dibasic phosphate based buffer, or a carbonate-hydrogen carbonate based buffer, so that the infusion preparation of the present invention can attain the buffering ability.

In the infusion preparation of the present invention, the above-described metal compounds, that is, the metal salts of weak acids or metal hydroxides, may be used alone or in combination as the source of basic metal ions, but preferably at least one metal salt of weak acid is used. When at least one metal salt of the weak acid is used, the infusion preparation of the present invention can attain the buffering ability against the fluctuations of pH.

In the infusion preparation of the present invention, the content of the above-described metal compound (metal salt of weak acids, metal hydroxide or the like) is not particularly limited, but it will be preferable that the content of the basic metal ion supplied by the metal compound is in the range of about 1 to about 5 equivalents, and more preferably about 1.2 to about 3.5 equivalents based on 1 equivalent of (2R)-2-propyloctanoic acid or a salt thereof

The infusion preparation of the present invention may further contain, in addition to (2R)-2-propyloctanoic acid or a salt as the active ingredient and substances as the source of the basic metal ion, one or at least two selected from electrolytes, saccharides, vitamins, protein amino acids, fat emulsions and the like. Preferably, it contains one or at least two selected from electrolytes, saccharides, vitamins, protein amino acids and the like. The components exemplified in the following as the electrolytes include some components which are identical with the components described as the metal salts of weak acids in the above. However, the components and contents thereof as described below as the electrolytes imply those required for the maintenance of the biological functions or the electrolytic balance in body fluids, and the purpose of addition thereof is different from that of the metal salts of weak acids described above as the source of basic metal ions. Therefore, the infusion preparation of the present invention may further contain, in addition to the above-described metal salts of weak acids, components identical with these as the electrolytes.

Examples of the electrolytes include sodium chloride, potassium chloride, calcium chloride, sodium lactate, sodium dihydrogen phosphate, magnesium carbonate and the like, and examples of the saccharides include glucose, fructose, sorbitol, mannitol, dextran and the like. Furthermore, examples of the vitamins may include vitamin B1 vitamin C and the like, and examples of the protein amino acids may include essential amino acids (for example, isoleucine, leucine, lysine, methionine, phenylalanine, threonine, tryptophan, valine, *etc*.), nonessential amino acids (for example, arginine, histidine, aminoacetic acid, aspartic acid, glutamic acid, alanine, cysteine, proline, serine, tyrosine, etc.) and the like. These optional components may be used alone or in combination, at any concentrations.

Other preferred substances which may be also contained in the infusion preparation of the present invention in addition to (2R)-2-propyloctanoic acid or a salt thereof which is the active ingredient are, for example, sodium chloride, glucose and the like.

As the contents of these substances, in the case of sodium chloride, for example, it is preferably equivalent to that of the saline, that is, 0.9%(w/v), and in the case of glucose, for example, it is preferably equivalent to that of a conventionally used sugar solution for infusion, that is, 5 to 70%(w/v), and, for example particularly preferably 5%(w/v) and 10%(wlv).

The electrolytes, saccharides, vitamins and protein amino acids are detailed below in detail.

For the electrolytes, it is possible to use various water-soluble salts which is conventionally used in infusion. For example, it is possible to use the water-soluble salts (for example, chloride, sulfate, acetate, gluconate, lactate, *etc*.) of various inorganic components (for example, sodium, potassium, calcium, magnesium, phosphorus, *etc*.), which are necessary in maintaining the biological functions and the electrolytic balance in body fluids. Such a water-soluble salt may be a solvate such as hydrate.

The water-soluble salts to provide the inorganic components described above include, for example, sodium: for example, sodium chloride, sodium lactate, sodium acetate, sodium sulfate, sodium glycerophosphate, *etc*.; potassium: for example, potassium chloride, potassium lactate, potassium acetate, potassium sulfate, potassium glycerophosphate, etc.; calcium: for example, calcium chloride, calcium lactate, calcium acetate, calcium glycerophosphate, calcium gluconate, calcium pantothenate, *etc*.; and magnesium: for example, magnesium sulfate, magnesium chloride, magnesium lactate, magnesium acetate, magnesium glycerophosphate, *etc.* As the source of phosphorus, the phosphoric acid esters of saccharides or salts thereof are used. Specific examples include glycerophosphoric acid, mannitol-1-phosphate, sorbitol-1-phosphate, glucose-6-phosphate, fructose-6-phosphate, mannose-6-phosphate and the like. In addition, the salts of these esters are preferably the alkali metal salts such as the sodium salts, potassium salts and the like. More specifically, the sodium salt and potassium salt of glycerophosphoric acid are preferred.

The contents of the electrolytes are not particularly limited, but the contents in about 1 liter of the infusion preparation of the present invention may be, for example, sodium: preferably about 15 to about 154 mEq; potassium: preferably about 2 to about 35 mEq; calcium: preferably about 2.5 to about 4.5 mEq; magnesium: preferably about 2 to about 5 mEq; phosphoric acid: preferably 3 to about 18 mEq, or the like.

For the saccharides, those that are conventionally used in various solutions can be used without particular limitation. Examples include monosaccharides such as glucose and fructose; disaccharides such as maltose; polyalcohols such as glycerol; sugar alcohols such as xylitol, sorbitol and mannitol; and dextrans such as dextran 40 and dextran 80. These may be used alone or may be also used in combination of two or more. The contents of the saccharides are not particularly limited, but preferably, for example, about 50 to about 500 g in about 1 liter of the infusion preparation of the present invention.

As the vitamins, various vitamins, including both the water-soluble vitamins and the fat-soluble vitamins, can be used without particular limitation. Examples include vitamin A, provitamin A, vitamin D, provitamin D, vitamin E, vitamin K, vitamin B1, vitamin B2, niacin, vitamin B6 group, pantothenic acid, biotin, myoinositol, choline, folic acid, vitamin B12, vitamin C, vitamin P, vitamin U and the like.

Upon addition of these vitamins, commercially available vitamin preparations which contain various vitamins may be used. Such preparations include, for example, "Multamin" (registered trademark) (trade name; manufactured by Sankyo) or the like. The contents of vitamins are not particularly limited, but the contents with respect to 1 liter of the infusion preparation of the present invention may be, for example, as follows: vitamin A: preferably about 2300 to about 3300 IU; vitamin D: preferably about 100 to about 400 IU; vitamin E: preferably about 5 to about 70 mg; vitamin K: preferably about 0.1 to about 3 mg; vitamin B1: preferably about 1.0 to about 50 mg; vitamin B2: preferably about 1.0 to about 10 mg; vitamin B6 group: preferably about 1 to about 15 mg; pantothenic acid: preferably about 4.5 to about 15 mg; biotin: preferably about 20 to about 300 µg; folic acid: preferably about 100 to about 1000 µg; vitamin B12: preferably about 1 to about 5 µg; vitamin C: preferably about 25 to about 500 mg, and the like. The infusion preparation of the present invention may also contain niacin, myoinositol, choline, vitamin P, vitamin U or the like, if necessary.

As the protein amino acids, various protein amino acids (essential amino acids, nonessential amino acids) which are contained in conventional amino acid solutions for alimentation and for the supply of the nitrogen source can be used without particular limitation.

Examples include L-isoleucine, L-leucine, L-valine, L-lysine, L-methionine, L-phenylalanine, L-threonine, L-tryptophan, L-arginine, L-histidine, glycine, L-alanine, L-proline, L-aspartic acid, L-serine, L-tyrosine, L-glutamic acid, L-cysteine and the like.

The protein amino acids are not necessarily used in the form of free amino acids, and they may be used in the form of inorganic acid salts (L-lysine hydrochloride, etc.), organic acid salts (L-lysine acetate, L-lysine malate, *etc*.), hydrolyzable esters *in vivo* (L-tyrosine methyl ester, L-methionine methyl ester, L-methionine ethyl ester, *etc*.), N-substituted form (N-acetyl-L-tryptophan, N-acetyl-L-cysteine, N-acetyl-L-proline, *etc.)* or the like.

Furthermore, they may be used in the form of dipeptides in which amino acids of the same species or of different species are peptide-bonded (L-tyrosyl-L-tyrosine, L-alanyl-L-tyrosin, L-arginyl-L-tyrosine, L-tyrosyl-L-arginine, *etc*.) or the like.

The blending proportions of such protein amino acids are not particularly limited, and preferably use is made of typically those mixtures having various ratios of essential amino acids to nonessential amino acids (so-called "E/N ratio") or having various ratios of essential amino acids to the entire amino acids (so-called "E/T ratio"), or those mixtures containing appropriate amounts of branched amino acids taking account of the ratios with respect to essential amino acids or nonessential amino acids, according to the conventional indices (the Vuj-N prescription, the FAO prescription or the FAO/WHO prescription based on the required amounts of essential amino acids, or the prescription based on the Fischer's ratio on the amino acid composition in blood plasma).

The contents of the protein amino acids are not particularly limited, but the contents with respect to 1 liter of the infusion preparation of the present invention may be, for example, as follows: L-isoleucine: preferably about 1.8 to about 12.5 g; L-leucine: preferably about 2.0 to about 12.5 g; L-valine: preferably about 1.3 to about 9.6 g; L-lysine hydrochloride: preferably about 2.6 to about 22.3 g; L-methionine: preferably about 1.0 to about 11.3 g; L-phenylalanine: preferably about 1.8 to about 12.8 g; L-threonine: preferably about 1.3 to about 6.5 g; L-tryptophan: preferably about 0.5 to about 7.0 g; L-arginine hydrochloride: preferably about 1.6 to about 14.5 g; L-histidine hydrochloride: preferably about 1.3 to about 8.1 g; glycine: preferably about 0.2 to about 7.0 g; L-alanine: preferably about 1.4 to about 8.2 g; L-proline: preferably about 0.9 to about 10.6 g; L-aspartie acid: preferably about 0.5 to about 6.3 g; L-serine: preferably about 0.7 to about 5.0 g; L-tyrosine: preferably about 0.05 to about 0.6 g; L-glutamic acid: preferably about 0.3 to about 6.5 g; L-cysteine: preferably about 0.06 to about 1.5 g; and the like.

The infusion preparation of the present invention may further contain trace elements as the electrolyte. The term "trace elements" as used herein means elements that improve various deficiency symptoms which may occur upon conducting infusion therapy, in particular hyperalimentation therapy, on a patient.

Examples of such trace elements include iron, copper, zinc, manganese, iodine, selenium, molybdenum, chromium, fluorine or the like. These trace elements may be used as a single species or in combination of two or more species, depending on the patient's condition. The contents of the trace elements are not particularly limited, but the contents with respect to 1 liter of the infusion preparation of the present invention may be, for example, as follows: iron: preferably about 0.9 to about 224 µmol, and more preferably about 9 to about 70 µmol; copper: preferably about 0.9 to about 55 µmol, and more preferably about 1 to about 10 µmol; zinc: preferably about 3.85 to about 210 µmol, and more preferably about 38.5 to about 61.5 µmol; manganese: preferably 0 to about 51 µmol, and more preferably about 1 to about 14.5 µmol; selenium: preferably about 0.025 to about 5.0 µmol, and more preferably about 0.25 to about 2.5 µmol: iodine: preferably 0 to about 11 µmol, and more preferably about 0.6 to about 1.1 µmol; and the like.

The infusion preparation of the present invention may also contain other elements such as chromium, molybdenum, cobalt and fluorine, if necessary. These trace elements are incorporated to the infusion preparation of the present invention by dissolving water-soluble compounds comprising these elements into water for injection or other aqueous components.

Examples of the water-soluble compounds which serve as the sources of the respective elements are as follows: iron source: iron sulfate, ferrous chloride, ferric chloride, iron gluconate, *etc*., copper source: copper sulfate, *etc*.; zinc source: zinc sulfate, zinc chloride, zinc gluconate, zinc lactate, zinc acetate, *etc*.; manganese source: manganese sulfate, *etc.;* and the like. Furthermore, iodine, selenium, molybdenum, chromium, fluorine and the like can be also incorporated in the infusion preparation of the present invention by using water-soluble compounds thereof

Furthermore, the above-described trace elements can be also incorporated in the infusion preparation of the present invention by using commercially available preparations comprising a plurality of trace elements. Specific examples of commercially available products are "Mineralin" (registered trademark) (trade name; manufactured by Nihon Pharmaceutical/Takeda Pharmaceutical) and "Elemenmic" (registered trademark) (trade name; manufactured by Ajinomoto Pharma) comprising iron, copper, zinc, manganese and iodine, or "Parmirin" (registered trademark) (trade name; manufactured by Nihon Pharmaceutical/Takeda Pharmaceutical) and "Elemate" (registered trademark) (trade name; manufactured by Ajinomoto Pharma) comprising iron, copper, zinc and iodine.

The infusion preparation of the present invention may further contain fat emulsions. For the fat emulsions, it is preferable to use an oil-in-water emulsion prepared by dispersing fats in water using emulsifying agents, The formulation of fat emulsions can be carried out according to conventional methods. As the fats, any fats which are known to be edible oil can be used without particular limitation,

Preferred fats include, for example, one or at least two fats selected from the group consisting of plant oils (for example, soybean oil, cottanseed oil, safflower oil, corn oil, olive oil, coconut oil, purple perilla oil, perilla oil, *etc*.), fish oil (for example, cod liver oil, *etc*.), medium-chain triglyceride (for example, Panasate (trade name; manufactured by NOF), ODO (trade name; manufactured by The Nisshin Oil Mills), *etc.),* and synthetic triglycerides (for example, triglycerides of conventional compositions such as 2-linoleoyl-l,3-dioctanoylglycerol (8L8), 2-linoleoyl-1,3-didecanoylglycerol (10L10), *etc.,* or structural lipids, *etc*.).

For preferred emulsifiers may be any emulsifiers, so long as they are used in pharmaceutical preparations. Emulsifiers include, for example, one or at least two emulsifiers selected from the group consisting of egg yolk phospholipids, hydrogenated egg yolk phospholipids, soybean phospholipids, hydrogenated soybean phospholipids, and nonionic surfactants (for example, Pluronic F68 (manufactured by Asahi Denka Co., Ltd.), HCO-60 (manufactured by Nihon Chemical), *etc*.).

A particularly preferable fat emulsion is a fat emulsion using soybean oil as the fat and egg yolk phospholipids as the emulsifier. The contents of the fat emulsions are not particularly limited, but for example, are in the range of about 10 to about 100 g for fats and about 0.5 to about 12 g for emulsifiers per liter of the infusion preparation of the present invention are preferred.

An appropriate example of the infusion preparation of the present invention is, for example, an infusion preparation which comprises about 1 to about 5 equivalents of a basic metal ion based on 1 equivalent of (2R)-2-propyloctanoic acid or a salt thereof; comprises at least one selected from a metal salt of phosphoric acid, a metal salt of carbonic acid and a metal salt of sulfurous acid, and optionally further comprises a metal hydroxide, as a source of the basic metal ion; and has a pH of about 8.4 to about 9,0, and the like.

Herein, preferred metal salts of phosphoric acid include, for example, trisodium phosphate, disodium hydrogen phosphate, sodium dihydrogen phosphate, or hydrates thereof; preferred metal salts of carbonic acid include, for example, sodium carbonate, sodium hydrogen carbonate, or hydrates thereof; and preferred metal salts of sulfurous acid include, for example, sodium sulfite, sodium hydrogen sulfite, or hydrates thereof. Metal salts of these acids comprising potassium instead of sodium are also preferred.

Furthermore, preferred metal hydroxides include sodium hydroxide, potassium hydroxide and the like. As described above, these metal hydroxides may used in mixtures. The preferred ranges of the contents of the respective metal salt and of the content of the metal hydroxide will be illustrated in the following, as expressed in the weight based on the number of moles of (2R)-2-propyloctanoic acid or a salt thereof. However, these are only exemplary values for the case of using compounds comprising sodium, and if compounds comprising potassium in place of sodium are used, the values should be appropriately modified in accordance with the molecular weight of the element. The contents of the sodium salts of phosphoric acid, carbonic acid or sulfurous acid based on 1 mole of (2R)-2-propyloctanoic acid or a salt thereof, that is, the contents of these salts corresponding to 1 to 5 equivalents are, for example, as follows: (1) about 54.7 g to about 273.2 g of trisodium phosphate; (2) about 71.0 g to about 354.9 g of disodium hydrogen phosphate; (3) about 120.0 g to about 600.0 g of sodium dihydrogen phosphate; (4) about 53.0 g to about 265.0 g of sodium carbonate; (5) about 84.0 g to about 420.0 g of sodium hydrogen carbonate; (6) about 53.0 g to about 265.0 g of sodium sulfite; (7) about 104.0 g to about 520.0 g of sodium hydrogen sulfite; and the like. These contents are all expressed as the weight of a non-solvate (for example, an anhydrate or the like). In the case of using a solvate (for example, hydrate, etc.) comprising one of these components, it is preferable that the weight of addition, as expressed as the weight deducted of the weight of solvent (for example, water, *etc.),* fall within the range of the weight of non-solvate as described above. When sodium hydroxide and/or potassium hydroxide is added, it is preferable to adjust the amount of the added metal salt of weak acid appropriately while being careful not to impair the buffering ability and not to depart from the preferable range of pH.

For example, in the case of an infusion preparation comprising (2R)-2-propyloctanoic acid, preferred examples of the infusion preparation comprising sodium hydroxide and/or potassium hydroxide include an infusion preparation comprising about 1 equivalent of the corresponding sodium hydroxide and/or potassium hydroxide and about 1 to about 4 equivalents of the corresponding metal salt of weak acid, for example, disodium hydrogen phosphate, sodium dihydrogen phosphate, sodium hydrogen carbonate, sodium hydrogen sulfite, dipotassium hydrogen phosphate, potassium dihydrogen phosphate, potassium hydrogen carbonate, potassium hydrogen sulfite or the like, based on about 1 equivalent of (2R)-2-propyloctanoic acid.

In addition, it is also possible, by adding at least a certain amount of, for example, tris(hydroxymethyl)aminomethane instead of the above-described metal salts of weak acid, to provide an infusion preparation having the same effect, that is, to provide an infusion preparation comprising (2R)-2-propyloctanoic acid or a salt thereof, which is resistant to the fluctuation of pH.

The infusion preparation of the present invention includes all of those infusion preparations comprising (2R)-2-propyloctanoic acid or a salt thereof and basic metal ions (preferably, about 1 to about 5 equivalents of the basic metal ion based on 1 equivalent of (2R)-2-propyloctanoic acid or a salt thereof). The content of (2R)-2-propyloctanoic acid or a salt thereof which is the active ingredient is not limited, but for example, an infusion preparation containing about 0.01 to about 20 mg of (2R)-2-propyloctanoic acid or a salt thereof which is the active ingredient, and preferably about 0.1 to about 20 mg, per mL of the preparation, in terms of the quantity of (2R)-2-propyloctanoic acid.

Since the infusion preparation of the present invention is a preparation for intravenous administration, the osmotic pressure and pH of the preparation are preferably selected within the ranges that do not have adverse effect on the living body. Such range of the osmotic pressure may be, for example, about 0.8 to about 10, more preferably about 0.9 to about 6, and particularly preferably about 1 to about 2, or the like. Furthermore, the range of pH may be, for example, about 3.0 to about 10.0, more preferably about 4.0 to about 9.0, and particularly preferably about 5.0 to about 9.0, or the like.

The infusion preparation of the present invention can be produced by dissolving (2R)-2-propyloctanoic acid or a salt thereof in water (for example, water for injection, *etc.),* if necessary, together with each of the above-described component. The dissolution can be carried out at any order. Also, it can be produced by dissolving (2R)-2-propyloctanoic acid or a salt thereof having a high concentration by using an aqueous diluent such as commercially available infusions, such as amino acid complex infusions (for example, Aminoleban (manufactured by Otsuka), Amizet B (manufactured by Tanabe), Amizet XB (manufactured by Tanabe), Amiparen (manufactured by Otsuka), Neoamiyu (manufactured by Ajinomoto Pharma), Pleamin-P (manufactured by Fuso), Proteamin 12X (manufactured by Tanabe), Molipron-F (manufactured by Ajinomoto Pharma), *etc*.), sugar/electrolyte/amino acid solutions for high-calorie infusions (for example, PNTWIN (manufactured by Ajinomoto Pharma), Unicaliq (manufactured by Terumo-Tanabe), *etc*.), electrolyte infusions (for example, saline, lactate Ringer's solution (for example, Solita (manufactured by Shimizu), Solulact (manufactured by Terumo), Hartmann (manufactured by Koyabashi Seiyaku), Lactec (manufactured by Otsuka), *etc*.), glucose-reinforced lactate Ringer's solutions (for example, SolulacT D (manufactured by Terumo), Hartmann D (manufactured by Kobayashi Seiyaku), Lactec D (manufactured by Otsuka), *etc*.), glucose-reinforced acetate Ringer's solutions (for example, Veen-D (manufactured by Nikken Chemical), *etc*.), sorbitol-reinforced lactate Ringer's solutions (for example Solita S (manufactured by Shimizu), Lactec G (manufactured by Otsuka), *etc*.), maltose-reinforced lactate Ringer's solutions (for example Solulact TMR (manufactured by Terumo), Potacol R (manufactured by Otsuka), *etc*.), maltose-reinforced acetate solutions (for example, Actit (manufactured by Nikken Chemical), *etc*.), EL3 (maintenance fluid for adults; manufactured by Ajinomoto Pharma), 10% EL3 (maintenance fluid; manufactured by Ajinomoto Pharma), EN Supplementary Fluid (1A, 1B, 2A, 2B, 3A, 3B, 4A, 4B; manufactured by Otsuka), Solita T (Nos. 1, 2, 3, 3-G, 4; manufactured by Shimizu), Physiosol (manufactured by Otsuka), Soldem (1, 2, 3, 4, 5, 6; manufactured by Terumo), *etc*.), sugar/electrolyte for high-calorie infusions (for example, Triparen (Nos. 1, 2; manufactured by Otsuka), Hicaliq (No.1, NC-L, No.2, NC-N, 3, NC-H; manufactured by Terumo), Hicaliq RF (manufactured by Terumo), *etc*.), sugar/electrolyte/amino acid solutions for high-calorie infusions (for example, PNTWIN (Nos. -1, -2, -3; manufactured by Ajinomoto Pharma), Unicaliq (L, N; manufactured by Terumo-Tanabe), *etc*.) and the like.

A preferred process for producing the infusion preparation of the present invention is, in view of the convenience in pH control or the like, a process in which a high concentration of (2R)-2-propyloctanoic acid or a salt thereof is diluted. Specifically, mention may be made of for example, a process in which a metal salt of weak acid as described above is dissolved, at an amount of about 1 to about 5 equivalents based on 1 equivalent of (2R)-2-propyloctanoic acid or a salt thereof, optionally together with a metal hydroxide, in an aqueous solvent (for example, water, *etc*.), in order to provide a solution containing about 2.5 to about 100 mg/mL of (2R)-2-propyloctanoic acid or a salt thereof in terms of the quantity of (2R)-2-propyloctanoic acid, with the pH of the solution being about 8.4 to about 9.0, subsequently the resulting solution is diluted in a solution containing one or at least two selected from (1) electrolytes, (2) saccharides, (3) vitamins, and (4) protein amino acids, to a concentration of about 0.01 to about 20 mg/mL, and preferably about 0.1 to about 20 mg/mL in terms of the quantity of (2R)-2-propyloctanoic acid, and then the diluted solution is charged into a container for infusion preparations.

As specific examples of the solutions containing one or at least two selected from (1) electrolytes, (2) saccharides, (3) vitamins and (4) protein amino acids, mention may be made of the above-listed, generally commercially available solutions and the like. Among these generally commercially available solutions, preferred aqueous diluting agents for the infusion preparation of the present invention include, for example, electrolyte solutions (in particular, those not containing divalent metals such as magnesium, calcium or the like are preferred), sugar solutions and the like, and more preferred are the solutions of sodium chloride, glucose and the like. The contents of these substances are such that the content of sodium chloride is preferably, for example, equivalent to the concentration of saline, that is, 0.9%(w/v), and the content of glucose is preferably, for example, equivalent to the concentration of generally used sugar solutions for infusion preparations, that is, 5 to 70%(w/v), with 5%(w/v) and 10%(w/v) being particularly preferred.

The infusion preparation of the present invention can be prepared by filling the solution for infusion preparation as formulated according to the process described above into a container for infusion preparation. The filled amount to the container for infusion is not particularly limited. For example, it is preferable to fill one unit form, that is, one container such as bag, bottle or the like, to about 50 mL, about 100 mL, about 200 mL, about 250 mL, about 500 mL or about 1000 mL; it is more preferable to fill 1 unit form to about 100 mL, about 250 mL, about 500 mL or about 1000 mL; and it is particularly preferable to fill 1 unit form to about 500 mL or 1000 mL. Filling and sealing of the solution for infusion preparation in a container for infusion can be carried out by conventional methods. For example, use is made of a process in which the solution for infusion preparation which has been preliminarily sterilized by a sterilization operation such as filtration sterilization, thermal sterilization or the like, is aseptically charged and sealed in a container for infusion which has been separately sterilized according to a sterilization procedure such as electron beam sterilization, ultraviolet ray sterilization, γ-ray sterilization, high pressure steam sterilization, gas sterilization or the like; a process in which the solution for infusion preparation is charged and sealed in a container for infusion, and then the container for infusion with its content are sterilized according to the conventional procedures (for example, high pressure steam sterilization, hot water immersion sterilization, hot water shower sterilization, *etc*.); or the like. Furthermore, if desired, an operation such as filtration on a dust-free filter (for example, 0.45 µm methylcellulose membrane, *etc*.), prior to the charging into such a container. A preferred sterilization treatment for the infusion preparation of the present invention is the high pressure steam sterilization. High pressure steam sterilization is preferably carried out, for example, at 100 to 125°C for 5 to 30 minutes.

The container for infusion into which the infusion preparation of the present invention is charged may be a container generally used for infusion preparations.

Specifically, use is made of fluid bags made of plastics, fluid bottles made of plastics, fluid bottles made of glass or the like, as described above. There is no particular limitation on the shape and size of such a container. The capacity of the container for infusion may be, for example, 50 mL, 100 mL, 200 mL, 250 mL, 500 mL, 1000 mL or the like, and 500 mL or 1000 mL is highly preferred. The material for the container for infusion is preferably a glass material or a plastic material which is compatible with the infusion preparation.

For the plastic material, flexible resins which have been conventionally used in container for infusions are preferred. More preferred resins may include soft synthetic resins having the resistance to heat to a certain degree (polyolefins (polyethylene, polypropylene, ethylene-propylene copolymers, mixtures of polypropylene with polyethylene or polybutene, partially crosslinked products of the polyolefins, ethylene-vinyl acetate copolymers, ethylene-(meth)acrylate copolymers, ethylene-(meth)acrylic acid copolymers, ethylene-maleic anhydride copolymers, *etc*.), polyvinyl chloride, vinyl chloride-vinyl acetate copolymers, ethylene fluoride-vinylidene chloride copolymers, polyesters (polyethylene terephthalate, polybutylene terephthalate), nylon, styrene-based elastomers (styrene-ethylene-butylene-styrene block copolymers, hydrogenated styrene-ethylene-butadiene copolymers, hydrogenated styrene-isoprene-styrene copolymers, *etc.), etc*.) and the like.

In the process for producing the infusion preparation of the present invention, when the contents in the container are vigorously foamed so that it requires a long time to obtain a clear solution, it may be attempted to reduce the time taken by using silicone-coated container for infusions.

For the silicones used in the coating, mention may be made of silicone oils (for example, dimethylpolysiloxane, methyl hydrogen polysiloxane, *etc*.), silicone varnish (for example, methyl silicone varnish, methyl phenyl silicone varnish, *etc*.) or the like, and a preferred example of silicone is KM-740 (manufactured by Shin-Etsu Chemical).

Among the infusion preparation of the present invention, in particular an infusion preparation showing alkalinity may occasionally undergo generation of insoluble allotrio when stored in a container made of glass material. When such infusion preparation is charged into a container for infusion made of glass material, generation of insoluble allotrio can be suppressed by coating the inner surface of the container with silicones or by treating the inner surface with silicon dioxide (for example, silicoat, *etc*.), and thus it is possible to provide an infusion preparation showing no problem in the generation of insoluble allotrio even under long-term storage. The silicone coating is carried out using the above-described silicones or silicone-based coating agents (for example, dimethyl silicone oil, methyl phenyl silicone oil, methyl hydrogen silicone oil, *etc*.), on the inner surfaces of such a container to a coating thickness of about 100 µm or less, and preferably about 15 to about 50 µm, by conventional methods, for example, thermal evaporation, plasma-enhanced chemical vapor deposition, pulsed-plasma chemical vapor deposition or the like. The treatment with silicon dioxide is carried out by conventional methods, for example, silicoat treatment, wave plasma chemical vapor deposition treatment or the like. Furthermore, when a plastic container is used, there is no problem in the occurrence of insoluble allotrio, and it is possible to provide a pharmaceutical preparation which has no problem in generation of insoluble allotrio even under long-term storage, without any treatment.

The infusion preparation of the present invention which is provided as charged in the above-described container for infusion can be directly administered, in a portion or in the whole amount, by intravenous drip administration to the veins of a patient. Furthermore, if desired, it is also possible to use the preparation as a mixture with other medicaments (for example, antibiotics, *etc*.) or in combination with them.

### Application for medicaments:

The infusion preparation of the present invention which contains (2R)-2-propyloctanoic acid or a salt thereof is useful for the treatment and/or prevention of, for example, neurodegenerative diseases of mammals (for example, human, non-human animals such as monkey, sheep, cow, horse, dog, cat, rabbit, rat, mouse, *etc*.). Neurodegenerative diseases include, for example, Alzheimer's disease, Parkinson's disease, amyotrophic lateral sclerosis, progressive supranuclear palsy, olivopontocerebellar atrophy, cerebral stroke (for example, cerebral infarction, cerebral hemorrhage, *etc*.), or neurofunctional disorders after neurospinal trauma (for example, demyelinating diseases (multiple sclerosis, *etc*.), brain cancer (astrocytoma, *etc*.), cerebrospinal diseases associated with infection (meningitis, pyocephalus, Creutzfeldt-Jakob disease, AIDS (HIV) dementia, *etc*.)) and the like. Furthermore, the present medicament is useful as a nerve regeneration promoter, an S100β increase inhibitor, or a nerve disorder improver. The infusion preparation of the present invention is intravenously administered into the living body under the purpose of treatment and/or prevention of the above-described diseases.

The daily dosage of the infusion preparation depends on the severity of the symptoms, age, sex, or body weight of the patient; the timing or the interval of administration; the type of the active ingredient or the like, and, without being limited, for example, in the case of intravenously administering it as an agent for treating neurodegenerative diseases including cerebral infarction, it is preferable, to set the daily dose of about 2 to about 12 mg per kg of the weight of the patient, when (2R)-2-propyloctanoic acid is used as the active ingredient. More preferably, the dosage is about 2 mg, about 4 mg, about 6 mg, about 8 mg, about 10 mg, about 12 mg or the like per kg of the patient's weight per day. Particularly preferably, the daily dose is about 4 mg, about 6 mg, about 8 mg or about 10 mg per kg of the patient's weight, and the daily dose is in particular suitable at about 4 mg or about 8 mg per kg of the patient's weight. Furthermore, when a salt of (2R)-2-propyloctanoic acid is used as the active ingredient, the daily doses as described above in terms of (2R)-2-propyloctanoic acid, are preferred.

The infusion preparation of the present invention may be also combined with other medicaments, for example, an anticonvulsant (for example, phenobarbital, mephobarbital, metharbital, primidone, phenytoin, ethotoin, trimethadione, ethosuximide, acetylphenetride, carbamazepine, acetazolamide, diazepam, sodium valproate, *etc*.), an acetylcholinesterase inhibitor (for example, donepezil hydrochloride, TAK-147, rivastigmine, galantamine, *etc*.), a neurotrophic factor (for example, ABS-205, *etc*.), an aldose reductase inhibitor, an antithrombotic (for example, t-PA, heparin, an oral anticoagulant (for example, warfarin, *etc.),* a synthetic antithrombin drug (for example, gabexate mesylate, nafamostat mesylate, argatroban, *etc*.), an antiplatelet drug (for example, aspirin, dipyridamole, ticlopidine hydrochloride, beraprost sodium, cilostazol, sodium ozagrel, *etc*.), a thrombolytic agent (for example, urokinase, tisokinase, alteprase, *etc.),* a Factor Xa inhibitor, a Factor VIIa inhibitor, a cerebral blood flow and metabolism improver (for example, idebenone, calcium hopantenate, amantadine hydrochloride, medofenoxate hydrochloride, dihydroergotoxine mesylate, pyrithioxin hydrochloride, γ-aminobutyric acid, bifemelane hydrochloride, lisuride maleate, indeloxazine hydrochloride, nicergoline, propentofylline, *etc.),* an antioxidant (for example, edaravone, *etc*.), a glycerin preparation (for example, glyceol, *etc.),* a β-secretase inhibitor (for example, 6-(4-biphenylyl)methoxy-2-[2-(N,N-dimethylamino)ethyl]tetraline, 6-(4-biphenylyl)methoxy-2-(N,N-dimethylamino)methyltetraline, 6-(4-biphenylyl)methoxy-2-(N,N-dipropylamino)methyltetraline, 2-(N,N-dimethylamino)methyl-6-(4'-methaxybiphenyl-4-yl)methoxytetraline, 6-(4-biphenylyl)methoxy-2-[2-(N,N-diethylamino)ethyl]tetraline, 2-[2-(N,N-dimethylamino)ethyl]-6-(4'-methylbiphenyl-4-yl)methoxytetraline, 2-[2-(N,N-dimethylamino)ethyl]-6-(4'-methoxybiphenyl-4-yl)methoxytetraline, 6-(2',4'-dimethoxybiphenyl-4-yl)methoxy-2-[2-(N,N-dimethylamino)ethyl]tetraline, 6-[4-(1,3-benzodioxol-5-yl)phenyl]methoxy-2-[2-(N,N-dimetbylamino)ethyl]tetraline, 6-(3',4'-dimethoxybiphenyl-4-yl)methoxy-2-[2-(N,N-dimethylamino)ethyl]tetraline, and optical isomers, salts and hydrates thereof, OM99-2 (WO 01/00663), *etc*.), a β-amyloid protein aggregation inhibitor (for example, PTI-00703, ALZHEMED (NC-531), PPI-368 (JP-T-11-514333), PPI-558 (JP-T-2001-500852), SKF-74652 *(Biochem. J*., 340(1), 283-289 (1999)), *etc*.), a cerebral function activator (for example, aniracetam, nicergoline, *etc*.), a dopamine receptor agonist (for example, L-dopa, bromocriptine, pergolide, talipexole, pramipexole, cabergoline, amantadine, *etc.),* a monoamine oxidase (MAO) inhibitor (for example, safrazine, deprenyl, selegiline, ramacemide, riluzole, *etc.),* a cholinergic blocking agent (for example, trihexyphenidyl, biperiden, *etc.),* a COMT inhibitor (for example, entacapone, *etc*.), a therapeutic agent for amyotrophic lateral sclerosis (for example, riluzole, a neurotrophic factor, *etc*.), a statin-based therapeutic agent for hyperlipidemia (for example, sodium pravastatin, atorvastatin, simvastatin, rosuvastatin, *etc*.), a fibrate-based therapeutic agent for hyperlipidemia (for example, clofilbrate, *etc*.), an apoptosis inhibitor (for example, CPI-1189, IDN-6556, CEP-1347, *etc.),* a nerve differentiation and regeneration promoter (for example, leteprinim, xaliproden (SR-57746-A), SB-216763, *etc*.), a non-steroidal anti-inflammatory drug (for example, meloxicam, tenoxicam, indomethacin, ibuprofen, celecoxib, rofecoxib, aspirin, indomethacin, *etc*.), a steroid drug (for example, dexamethasone, hexestrol, cortisone acetate, *etc*.), a sexual hormone or derivatives thereof (for example, progesterone, estradiol, estradiol benzoate, *etc*.), or the like.

Furthermore, it may be also combined with a nicotinic receptor regulator, a γ-secretase inhibitor, a β-amyloid vaccine, a β-amyloid cleaving enzyme, a squalene synthetase inhibitor, a therapeutic agent for the abnormal behavior, wandering or the like associated with progress of dementia, a hypotensor, a therapeutic agent for diabetes mellitus, an antidepressant, an antianxiety agent, a disease-modifying antirheumatoid agent, an anticytokine agent (for example, a TNF inhibitor, a MAP kinase inhibitor, *etc.),* a parathyroid hormone (PTH), a calcium receptor antagonist or the like. These combination medicaments are only exemplary and are not limited to these. Other medicaments may be administered in any combination of two or more. Furthermore, the medicaments for combined use include those that have been discovered as well as those that are to be discovered afterward, based on the mechanism described above.

### Toxicity:

Toxicity of (2R)-2-propyloctanoic acid or a salt thereof is very low, and it is considered to be sufficiently safe for the use as a pharmaceutical drug. For example, it was found that in a single intravenous administration using a dog, (2R)-2-propyloctanoic acid did not result in death at a dose of 100 mg/kg in any case.

### Effects of the Invention:

The present invention provides an infusion preparation for continuous intravenous administration containing (2R)-2-propyloctanoic acid or a salt thereof, which has a pH appropriate for intravenous administration, and which does not require any preparative operation such as dissolution, dilution or the like at the time of use, and a process for producing the same.

### Best Modes for Carrying Out the Invention

Hereinafter, the present invention will be explained by way of Examples, and the present invention is not intended to be limited to these. Furthermore, modifications may be made within the range of not departing from the scope of the present invention.

### Example 1-1

Trisodium phosphate dodecahydrate (7.08 kg), sodium chloride (18 kg) and (2R)-2-propyloctanoic acid (4.0 kg) were added to water for injection, which was further added to give a total volume of 2000 L. The mixture was made to a homogeneous solution and then filtered on an sterilizing filter (0.22 µm membrane manufactured by Durapore), and the resulting solution was filled into fluid bags (100 mL, 250 mL and 500 mL). These filled containers were capped and then autoclaved (123°C, 15 minutes) to prepare the infusion preparations described in Table 1 below. The dissolved state of each preparation was clear and colorless, and the pH was in the range of 5.0 to 9.0.

**Table 1**

| | Formulation | | |
|---|---|---|---|
| | 1 | 2 | 3 |
| (2R)-2-Propyloctanoic acid (mg) | 200 | 500 | 1000 |
| Trisodium phosphate·dodecahydrate (mg) | 354 | 885 | 1770 |
| Sodium chloride (mg) | 900 | 2250 | 4500 |
| Water for injection (in total) (mL) | 100 | 250 | 500 |

### Example 1-2

Trisodium phosphate·dodecahydrate (14.16 kg) and (2R)-2-propyloctanoic acid (8.0 kg) were added to water for injection and dissolved therein. Sodium chloride (18 kg) was added to the resulting solution, and water for injection was further added to give a total volume of 2000 L. The mixture was made to a homogeneous solution and then filtered on an sterilizing filter (0.22 µm membrane manufactured by Durapore), and the resulting solution was filled into fluid bags (100 mL, 250 mL and 500 mL). These filled containers were capped and then autoclaved (123°C, 15 minutes) to prepare the infusion preparations described in Table 2 below. The dissolved state of each preparation was clear and colorless, and the pH was in the range of 5.0 to 9.0.

**Table 2**

| | Formulation | | |
|---|---|---|---|
| | 4 | 5 | 6 |
| (2R)-2-Propyloctanoic acid (mg) | 400 | 1000 | 2000 |
| Trisodium phosphate·dodecahydrate (mg) | 708 | 1770 | 3540 |
| Sodium chloride (mg) | 900 | 2250 | 4500 |
| Water for injection (in total) (mL) | 100 | 250 | 500 |

### Example 2-1

Disodium hydrogen phosphate-dodecahydrate (6.4 kg), sodium chloride (18 kg) and (2R)-2-propyloctanoic acid (4.0 kg) were added to water for injection, which was further added to give a total volume of 2000 L. The mixture was made to a homogeneous solution and then filtered on an sterilizing filter (0.22 µm membrane manufactured by Durapore), and the resulting solution was filled into fluid bags (100 mL, 250 mL and 500 mL). These filled containers were capped and then autoclaved (123°C, 15 minutes) to prepare the infusion preparations described in Table 3 below. The dissolved state of each preparation was clear and colorless, and the pH was in the range of 5.0 to 9.0.

**Table 3**

| | Formulation | | |
|---|---|---|---|
| | 7 | 8 | 9 |
| (2R)-2-Propyloctanoic acid (mg) | 200 | 500 | 1000 |
| Disodium hydrogen phosphate dodecahydrate (mg) | 320 | 800 | 1600 |
| Sodium hydroxide (mg) | 41.2 | 103.0 | 206.0 |
| Sodium chloride (mg) | 900 | 2250 | 4500 |
| Water for injection (in total) (mL) | 100 | 250 | 500 |

### Example 2-2

Disodium hydrogen phosphate·dodecahydrate (12.8 kg) and (2R)-2-propyloctanoic acid (8.0 kg) were added to water for injection and dissolved therein. Sodium chloride (18 kg) was added to the resulting solution, and water for injection was further added to give a total volume of 2000 L. The mixture was made to a homogeneous solution and then filtered on an sterilizing filter (0.22 µm membrane manufactured by Durapore), and the resulting solution was filled into fluid bags (100 mL, 250 mL and 500 mL). These filled containers were capped and then autoclaved (123°C, 15 minutes) to prepare the infusion preparations described in Table 4 below. The dissolved state of each preparation was clear and colorless, and the pH was in the range of 5.0 to 9.0.

**Table 4**

| | Formulation | | |
|---|---|---|---|
| | 10 | 11 | 12 |
| (2R)-2-Propyloctanoic acid (mg) | 400 | 1000 | 2000 |
| Disodium hydrogen phosphate·dodecahydrate (mg) | 640 | 1600 | 3200 |
| Sodium hydroxide (mg) | 82.4 | 206.0 | 412.0 |
| Sodium chloride (mg) | 900 | 2250 | 4500 |
| Water for injection (in total) (mL) | 100 | 250 | 500 |

### Example 3-1

Disodium hydrogen phosphate·dodecahydrate (12.8 kg), sodium chloride (18 kg) and (2R)-2-propyloctanoic acid·sodium salt (8.944 kg) were added to water for injection, which was further added to give a total volume of 2000 L. The mixture was made to a homogeneous solution and then filtered on an sterilizing filter (0.22 µm membrane manufactured by Durapore), and the resulting solution was filled into fluid bags (100 mL, 250 mL and 500 mL). These filled containers were capped and then autoclaved (123°C, 15 minutes) to prepare the infusion preparations described in Table 5 below. The dissolved state of each preparation was clear and colorless, and the pH was in the range of 5.0 to 9.0.

**Table 5**

| | Formulation | | |
|---|---|---|---|
| | 13 | 14 | 15 |
| (2R)-2-Propyloctanoic acid·sodium salt (mg) | 447.2 | 1118.0 | 2236.0 |
| Disodium hydrogen phosphate·dodecahydrate (mg) | 640 | 1600 | 3200 |
| Sodium chloride (mg) | 900 | 2250 | 4500 |
| Water for injection (in total) (mL) | 100 | 250 | 500 |

### Example 3-2

Disodium hydrogen phosphate-dodecahydrate (6.4 kg), sodium chloride (18 kg) and (2R)-2-propyloctanoic acid·sodium salt (4.472 kg) were added to water for injection, which was further added to give a total volume of 2000 L. The mixture was made to a homogeneous solution and then filtered on an sterilizing filter (0.22 µm membrane manufactured by Durapore), and the resulting solution was filled into fluid bags (100 mL, 250 mL and 500 mL). These filled containers were capped and then autoclaved (123°C, 15 minutes) to prepare the infusion preparations described in Table 6 below. The dissolved state of each preparation was clear and colorless, and the pH was in the range of 5.0 to 9.0.

**Table 6**

| | Formulation | | |
|---|---|---|---|
| | 16 | 17 | 18 |
| (2R)-2-Propyloctanoic acid-sodium salt (mg) | 223.6 | 559.0 | 1118.0 |
| Disodium hydrogen phosphate-dodecahydrate (mg) | 320 | 800 | 1600 |
| Sodium chloride (mg) | 900 | 2250 | 4500 |
| Water for injection (in total) (mL) | 100 | 250 | 500 |

### Example 4-1

Disodium hydrogen phosphate·dodecahydrate (6.4 kg), glucose (100 kg) and (2R)-2-propyloctanoic acid (4.0 kg) were added to water for injection, which was further added to give a total volume of 2000 L. The mixture was made to a homogeneous solution and then filtered on an sterilizing filter (0.22 µm membrane manufactured by Durapore), and the resulting solution was filled into fluid bags (100 mL, 250 mL and 500 mL). These filled containers were capped and then autoclaved (123°C, 15 minutes) to prepare the infusion preparations described in Table 7 below. The dissolved state of each preparation was clear and colorless, and the pH was in the range of 5.0 to 9.0.

**Table 7**

| | Formulation | | |
|---|---|---|---|
| | 19 | 20 | 21 |
| (2R)-2-Propyloctanoic acid (mg) | 200 | 500 | 1000 |
| Disodium hydrogen phosphate dodecahydrate (mg) | 320 | 800 | 1600 |
| Sodium hydroxide (mg) | 41.2 | 103.0 | 206.0 |
| Glucose (g) | 5.0 | 12.5 | 25.0 |
| Water for injection (in total) (mL) | 100 | 250 | 500 |

### Example 4-2

Disodium hydrogen phosphate·dodecahydrate (12.8 kg) and (2R)-2-propyloctanoic acid (8.0 kg) were added to water for injection and dissolved therein. Glucose (100 kg) was added to the resulting solution, and water for injection was further added to give a total volume of 2000 L. The mixture was made to a homogeneous solution and then filtered on an sterilizing filter (0.22 µm membrane manufactured by Durapore), and the resulting solution was filled into fluid bags (100 mL, 250 mL and 500 mL). These filled containers were capped and then autoclaved (123°C, 15 minutes) to prepare the infusion preparations described in Table 8 below. The dissolved state of each preparation was clear and colorless, and the pH was in the range of 5.0 to 9.0.

**Table 8**

| | Formulation | | |
|---|---|---|---|
| | 22 | 23 | 24 |
| (2R)-2-Propyloctanoic acid (mg) | 400 | 1000 | 2000 |
| Disodium hydrogen phosphate dodecahydrate (mg) | 640 | 1600 | 3200 |
| Sodium hydroxide (mg) | 82.4 | 206.0 | 412.0 |
| Glucose (g) | 5.0 | 12.5 | 25.0 |
| Water for injection (in total) (mL) | 100 | 250 | 500 |

### Industrial Applicability

The present invention relates to an infusion preparation for continuous intravenous administration containing (2R)-2-propyloctanoic acid or a salt thereof, which has a pH appropriate for intravenous administration, and which does not require any preparative operation such as dissolution, dilution or the like at the time of use. The infusion preparation of the present invention is an excellent infusion preparation which is resistant to fluctuations of pH, which does not present the risk of clouding when jointly used with other medicaments or administered to blood containers of a patient, and which can be used safely, and thus it is applicable as a medicament.

## Claims

1. An infusion preparation comprising (2R)-2-propyloctanoic acid or a salt thereof and a basic metal ion.

2. The infusion preparation according to claim 1, which comprises at least one selected from a metal salt of phosphoric acid, a metal salt of carbonic acid, a metal salt of sulfurous acid, a metal salt of organic sulfonic acid and a metal salt of organic C2-6 carboxylic acid as a source of the basic metal ion, and optionally further comprises a metal hydroxide.

3. The infusion preparation according to claim 1, which further comprises one or at least two selected from (i) electrolytes, (ii) saccharides, (iii) vitamins and (iv) protein amino acids.

4. The infusion preparation according to claim 1, which comprises about 1 to about 5 equivalents of the basic metal ion based on 1 equivalent of (2R)-2-propyloctanoic acid or a salt thereof.

5. The infusion preparation according to claim 2, which comprises at least one selected from trisodium phosphate, disodium hydrogen phosphate, sodium dihydrogen phosphate, sodium carbonate, sodium hydrogen carbonate, sodium sulfite, sodium hydrogen sulfite, tripotassium phosphate, dipotassium hydrogen phosphate, potassium dihydrogen phosphate, potassium carbonate, potassium hydrogen carbonate, potassium sulfite and potassium hydrogen sulfite, and optionally further comprises sodium hydroxide and/or potassium hydroxide, as a source(s) of the basic metal ion.

6. The infusion preparation according to claim 2, which comprises sodium hydroxide and/or potassium hydroxide, and further comprises at least one selected from disodium hydrogen phosphate, sodium dihydrogen phosphate, sodium hydrogen carbonate, sodium hydrogen sulfite, dipotassium hydrogen phosphate, potassium dihydrogen phosphate, potassium hydrogen carbonate and potassium hydrogen sulfite, as sources of the basic metal ion.

7. The infusion preparation according to claim 1, which has a pH of about 5.0 to about 9.0.

8. The infusion preparation according to claim 1, which comprises about 0.1 to about 20 mg of (2R)-2-propyloctanoic acid or a salt thereof per mL.

9. A container for infusion which is filled with the infusion preparation depicted in claim 8 at about 50 mL, about 100 mL, about 200 mL, about 250 mL, about 500 mL or about 1,000 mL per one unit.

10. The infusion preparation according to claim 1, which comprises about 1 to about 5 equivalents of the basic sodium ion based on 1 equivalent of (2R)-2-propyloctanoic acid or a salt thereof; comprises at least one selected from a sodium salt of phosphoric acid and a sodium salt of carbonic acid as a source of the basic sodium ion, and optionally further comprises sodium hydroxide, as a source(s) of the basic sodium ion; and has a pH of about 5.0 to about 9.0.

11. The infusion preparation according to claim 10, which further comprises 0.9% (w/v) sodium chloride.

12. The infusion preparation according to claim 1, wherein the salt of (2R)-2-propyloctanoic acid is a sodium salt or a basic natural amino acid salt.

13. The infusion preparation according to claim 1, which is an agent for preventing and/or treating neurodegenerative diseases, nerve disorders or diseases in need of nerve regeneration.

14. A process for producing an infusion preparation comprising (2R)-2-propyloctanoic acid or a salt thereof and a basic metal ion, which comprises dissolving (2R)-2-propyloctanoic acid or a salt thereof, one or at least two selected from a metal salt of phosphoric acid, a metal salt of carbonic acid, a metal salt of sulfurous acid, a metal salt of organic sulfonic acid and a metal salt of C2-6 organic acid, and optionally metal hydroxide in an aqueous medium to thereby prepare a solution comprising about 2.5 to about 100 mg/mL of (2R)-2-propyloctanoic acid or a salt thereof and having a pH of about 8.4 to about 9.0; diluting the prepared solution with one or at least two selected from (i) electrolytes, (ii) saccharides, (iii) vitamins and (iv) protein amino acids to thereby adjust the concentration of (2R)-2-propyloctanoic acid or a salt thereof in the solution to about 0.1 to about 20 mg/mL; and filling a container for infusion with the diluted solution.

15. A method for preventing and/or treating neurodegenerative diseases, nerve disorders or diseases in need of nerve regeneration, which comprises administering an effective amount of the infusion preparation according to claim 1 to a mammal.

16. Use of the infusion preparation according to claim 1 for the manufacture of an agent for preventing and/or treating neurodegenerative diseases, nerve disorders or diseases in need of nerve regeneration.
